# EUROPEAN PATENT SPECIFICATION

(11) **EP 2 944 327 B1**
(45) Date of publication and mention of the grant of the patent: **30.08.2017**
(21) Application number: 13871055.3
(22) Date of filing: 27.11.2013
(51) Int. Cl.: A61L 2/10, B65B 55/04, B65B 55/08

(54) **ULTRAVIOLET STERILIZER**
ULTRAVIOLETTSTERILISATOR
STÉRILISATEUR ULTRAVIOLET

(30) Priority: 10.01.2013 JP 2013002942
(43) Date of publication of application: 18.11.2015
(73) Proprietor: Shikoku Kakoki Co.,Ltd., Itano-gun, Tokushima 771-0287 (JP)
(72) Inventor: NISHIO, Yoji, Itano-gun Tokushima 771-0287 (JP); ITOH, Yasumasa, Itano-gun Tokushima 771-0287 (JP)
(74) Representative: D Young & Co LLP
(86) International application number: PCT/JP2013/006970
(87) International publication number: WO 2014/108953

(56) References cited:
- EP-A1- 0 634 883
- EP-A1- 1 956 608
- EP-A2- 1 721 684
- JP-A- 2000 506 822
- JP-A- 2008 500 303
- JP-A- 2012 085 850
- JP-U- H0 242 642
- US-A1- 2013 068 964

## Description

### Technical Field

The present invention relates to an ultraviolet sterilizer that sterilizes, e.g., a food packaging material by applying ultraviolet light (UV) to the food packaging material via an ultraviolet transmitting plate, and more specifically relates to an ultraviolet sterilizer capable of detecting breakage of the ultraviolet transmitting plate.

### Background Art

In the food industry, sterilization using an ultraviolet lamp device is used for, e.g., expiration date extension. In such ultraviolet lamp device, typically, quartz glass that transmits ultraviolet light having a particular wavelength (254 nm) is used in order to isolate a lamp provided inside the housing from the outside. In the entire industrial world, recent years, there has been an increasing demand for safety in various aspects from the perspective of product liability, and there have been cases where even use of glass products is not allowed for fear of contamination caused by breakage of glass; however, the current state is that there is a strong demand for use of quartz glass to bring satisfaction to functions of the ultraviolet lamp device, and thus, it is necessary to take some kind of measure for safety ensuring.

Under such circumstances as mentioned above, several methods for detecting breakage of an ultraviolet transmitting plate of quartz glass have been proposed; however, each of such methods have drawbacks and advantages, requiring further improvement and differentiation. For example, a method in which the inside of a housing is maintained at a medium pressure (approximately 0.1 MPa) using a cooling gas to detect a decrease in pressure and thereby monitor whether cracking of quartz glass occurs has been proposed (see Patent Document 1). However, in this method, there is a limit to detection of small breaks (cracks), and furthermore, the housing, which requires maintenance such as lamp replacement, is sealed itself, and thus, leakage may occur from a gap around packing or the like, and it is difficult to identify the source of the leakage, making it impossible to ensure reliable detection of quartz glass breakage.

Also, a method in which a wiring is bonded to an end part of quartz glass (a part of the quartz glass other than an effective part for ultraviolet light) via a special resin and current is applied to the wiring to detect disconnection upon breakage of the glass (see Patent Document 2) has been proposed, but such method has not yet actually employed in high-pressure sterilizers, and is low in reliability because the disconnection cannot be detected until the electric wire is completely ruptured and a crack in quartz glass thus may not be detected depending on the position of the crack or the degree of the breakage. Furthermore, it is very difficult to wire an entire surface of the quartz glass, which is unrealistic.

Also, a method in which a Teflon (registered trademark)-based special polymer film is disposed below quartz glass to prevent a drop of foreign matter upon breakage of the quartz glass or a UV lamp has been proposed (see Patent Document 3), but the method has problems of both a short lifetime of ultraviolet lamps of the high pressure mercury type because of exposure to high heat and substantially low transmittivity of the ultraviolet light compared to cases where the quartz glass is used alone.

Also, a method in which two ultraviolet transmitting plates such as quartz glass plates are arranged at an opening portion on the lower side of a housing, an enclosed space is formed between these ultraviolet transmitting plates, air of a minute pressure is introduced to the enclosed space or air is suctioned from the enclosed space, and breakage of an ultraviolet transmitting plate is detected when a flow sensor detects a flow of the air has been proposed (see Patent Document 4); however, applied ultraviolet light needs to pass through the two ultraviolet transmitting plates, resulting in an increase in attenuation rate to be approximately twice that of the case where a single ultraviolet transmitting plate is used. This problem can be solved by employment of a high-pressure mercury lamp that provides intense ultraviolet light; however, high-pressure mercury lamps are expensive, approximately 2 to 2.5 times the prices of low-pressure mercury lamps, and thus, have a drawback in terms of cost. On the other hand, where a low-pressure mercury lamp, which is inexpensive, is used, such lamp provides relatively-less intense ultraviolet light, thus causing a problem of incapability of providing a predetermined sterilization effect when the end of the lifetime of the lamp comes close. Furthermore, the method in Patent Document 4 also brings concern that if the gauge pressure (positive pressure or negative pressure) of the enclosed space between the ultraviolet transmitting plates is made excessively high, the ultraviolet transmitting plates may be broken.

Also, Patent Document 5 relates to a sports lighting fixture having a broken glass detection arrangement. It does not relate to an airflow detecting means comprising an air pressure-adjusting means inside an enclosed space and a flowmeter for detecting breakage of an ultraviolet transmitting plate.

### Prior Art Documents

### Patent Documents

Patent Document 1: Japanese Patent No. 3963480
Patent Document 2: Japanese Unexamined Patent Application Publication No. 2001-97319
Patent Document 3: Japanese Unexamined Patent Application Publication (Translation of PCT Application) No. 2005-519816
Patent Document 4: Japanese Unexamined Patent Application Publication No. 2012-85850
Patent Document 5: European Withdrawn Patent Application Publication No. 0 634 883 A1

### Summary of the Invention

### Object to be Solved by the Invention

An object of the present invention is to provide an ultraviolet sterilizer capable of detecting breakage of an ultraviolet transmitting plate of, e.g., quartz glass with high accuracy while suppressing attenuation of ultraviolet light by the ultraviolet transmitting plate.

### Means to Solve the Object

Under the circumstances stated in the Background Art section, the present inventors conduct diligent studies to obtain an ultraviolet sterilizer capable of detecting breakage of an ultraviolet transmitting plate of, e.g., quartz glass with high accuracy while suppressing attenuation of ultraviolet light by the ultraviolet transmitting plate. As a result of focusing on the fact that upon a crack being generated in an ultraviolet transmitting plate of, e.g., quartz glass, the crack reaches an end part of the ultraviolet transmitting plate even if the crack is a slight one and conducting further diligent studies, the present inventors found that, rather than arranging two ultraviolet transmitting plates to form an enclosed space therebetween as in the device described in Patent Document 4, forming an enclosed space at an outer peripheral part of a single ultraviolet transmitting plate enables detecting breakage of the ultraviolet transmitting plate with high accuracy while suppressing attenuation of ultraviolet light by the ultraviolet transmitting plate and thereby has completed the present invention. In other words, the present inventors found that according to the present invention, not two ultraviolet transmitting plates, but a single ultraviolet transmitting plate can be used, enabling suppression of attenuation of ultraviolet light by the ultraviolet transmitting plate, and furthermore, an enclosed space is formed at an outer peripheral part of the ultraviolet transmitting plate, enabling the gauge pressure of the enclosed space to be maintained higher or lower while avoiding the fear of breakage of the ultraviolet transmitting plate, and as a result, even if a fine crack is generated in the ultraviolet transmitting plate because of some abnormality, the amount of air flowed from or into the enclosed space increases, facilitating detection of the flow of the air in the enclosed space by a flowmeter, enabling detecting breakage of the ultraviolet transmitting plate with high accuracy.

In other words, the present invention relates to:
(1) An ultraviolet sterilizer comprising: an ultraviolet lamp; a housing surrounding the ultraviolet lamp; an ultraviolet transmitting section provided at an opening portion on a lower side of the housing, the ultraviolet transmitting section including an ultraviolet transmitting plate and an ultraviolet transmitting plate-holding means; and an air flow detecting means, wherein the ultraviolet transmitting section-holding means comprises a sealing means for forming an enclosed space which one end is in communication with the air flow detecting means, cooperating with an outer peripheral part of the ultraviolet transmitting plate, the air flow detecting means comprises an air pressure-adjusting means for adjusting an air pressure inside the enclosed space, and a flowmeter, and wherein a flow of air inside the enclosed space generated when the enclosed space is released from a sealed state due to breakage of the ultraviolet transmitting plate, is detected by the flowmeter;
(2) The ultraviolet sterilizer according to (1) above, wherein the outer peripheral part of the ultraviolet transmitting plate is an outer peripheral end surface or an outer peripheral edge part of the ultraviolet transmitting plate;
(3) The ultraviolet sterilizer according to (1) or (2) above, wherein the ultraviolet transmitting plate-holding means comprises a base plate fixed to a lower end of a side wall of the housing, and a retainer plate fixed on the base plate;
(4) The ultraviolet sterilizer according to (3) above, wherein the sealing means is a means for directly sealing a part of the base plate and a part of the retainer plate which are outward of the outer peripheral end surface of the ultraviolet transmitting plate; and sealing the base plate and the retainer plate via an outer peripheral edge part of the ultraviolet transmitting plate, and wherein an enclosed space is formed along an outer side of the outer peripheral end surface of the ultraviolet transmitting plate;
(5) The ultraviolet sterilizer according to (3) above, wherein the sealing means is a means for sealing the base plate and the retainer plate via two parts in the outer peripheral edge part of the ultraviolet transmitting plate, the two parts having a predetermined interval therebetween, and an enclosed space is formed as a recess peripheral groove of the retainer plate that corresponds to an upper surface side of the outer peripheral edge part of the ultraviolet transmitting plate and/or a recess peripheral groove of the base plate that corresponds to a lower surface side of the outer peripheral edge part of the ultraviolet transmitting plate;
(6) The ultraviolet sterilizer according to any one of (1) to (5) above, wherein the sealing means is an elastic body having an O-ring shape;
(7) The ultraviolet sterilizer according to any one of (1) to (6) above, wherein the air pressure adjusting means is an air suction means for suctioning air having a predetermined pressure from the enclosed space to maintain the enclosed space in a negative pressure state; and
(8) The ultraviolet sterilizer according to any one of (1) to (7) above, wherein the ultraviolet transmitting plate is made of quartz glass.

### Effect of the Invention

According to the present invention, rather than arranging two ultraviolet transmitting plates to form an enclosed space between the ultraviolet transmitting plates, forming an enclosed space at an outer peripheral part of a single ultraviolet transmitting plate enables the gauge pressure of the enclosed space to be maintained higher or lower while avoiding the fear of breakage of the ultraviolet transmitting plate, and as a result, even if a fine crack is generated in the ultraviolet transmitting plate because of some abnormality, the amount of air flowed from or into the enclosed space increases, enabling detecting the breakage of the ultraviolet transmitting plate with high accuracy. Also, the present invention enables use of a single ultraviolet transmitting plate and thus enables suppression of attenuation (illuminance loss) of ultraviolet light emitted by an ultraviolet lamp compared to cases where an enclosed space is formed by two ultraviolet transmitting plates as well as suppression of manufacturing costs. Furthermore, an ultraviolet sterilizer according to the present invention is not so complex in structure itself, and thus, the present invention can be applied to any of various types of ultraviolet sterilizers at low costs.

### Brief Description of Drawings

[Figure 1] Figure 1 is a schematic vertical cross-sectional view (front view) of an ultraviolet sterilizer according to the present invention.
[Figure 2] Figure 2 is an enlarged view of part A in Figure 1.
[Figure 3] Figure 3 is a partial enlarged view of an ultraviolet sterilizer according to another mode of the present invention.

### Mode of Carrying Out the Invention

An ultraviolet sterilizer according to the present invention is not specifically limited as long as such ultraviolet sterilizer is a ultraviolet sterilizer comprising: an ultraviolet lamp; a housing surrounding the ultraviolet lamp; an ultraviolet transmitting section provided at an opening portion on a lower side of the housing, the ultraviolet transmitting section including an ultraviolet transmitting plate and an ultraviolet transmitting plate-holding means; and an air flow detecting means, wherein the ultraviolet transmitting section-holding means comprises a sealing means for forming an enclosed space which one end is in communication with the air flow detecting means, cooperating with an outer peripheral part of the ultraviolet transmitting plate, the air flow detecting means comprises an air pressure-adjusting means for adjusting an air pressure inside the enclosed space, and a flowmeter, and wherein a flow of air inside the enclosed space generated when the enclosed space is released from a sealed state due to breakage of the ultraviolet transmitting plate, is detected by the flowmeter. The outer peripheral part of the ultraviolet transmitting plate is a collective name for an outer peripheral edge part and an outer peripheral end surface of the ultraviolet transmitting plate, and the outer peripheral edge part refers to a region of an upper surface and/or a lower surface of the ultraviolet transmitting plate near the outer peripheral end (normally, a region that is 2 to 100 mm from the outer peripheral end) of the ultraviolet transmitting plate in plan view, and the outer peripheral end surface refers to a region between two outer peripheral end (side peripheral surface) of the ultraviolet transmitting plate in side view.

For the ultraviolet lamp, a known ultraviolet lamp can arbitrarily be used in consideration of, e.g., an ultraviolet transmittance of the ultraviolet transmitting section and/or an amount of ultraviolet light required for a sterilization target. More specifically, any of ultraviolet lamps including low-pressure ultraviolet lamps, medium-pressure ultraviolet lamps and high-pressure ultraviolet lamps can be used regardless of whether a pressure of vapor in the ultraviolet lamp during the ultraviolet lamp being on is high or low. For obtaining a higher sterilization effect, it is preferable to use a high-pressure ultraviolet lamp such as a high-pressure mercury lamp or a metal halide lamp; however, the ultraviolet sterilizer according to the present invention enables use of a single ultraviolet transmitting plate, providing a low attenuation rate of ultraviolet light, and thus even use of a low-pressure ultraviolet lamp or a medium-pressure ultraviolet lamp is favorable. Also, a shape of the ultraviolet lamp used in the ultraviolet sterilizer according to the present invention is preferably a long linear tube shape or an elongated U-tube shape with a direction of movement of a sterilization target as a longitudinal direction thereof from the perspective of ensuring a certain length of time for sterilizing a sterilization target, and one or more such ultraviolet lamps may be provided. Also, from the perspective of more efficiently applying ultraviolet light from the ultraviolet lamp to a sterilization target, it is preferable to provide a reflective plate near the ultraviolet lamp.

The housing is not specifically limited in, e.g., shape and material as long as the housing surrounds the ultraviolet lamp in the present invention and includes an opening portion for the ultraviolet transmitting section on the lower side thereof. A favorable example of the shape of the housing can be a shape resulting from one side of a rectangular parallelepiped shape having six sides being removed to form the lower-side opening portion for the ultraviolet transmitting section. Also, the housing may be formed integrally with the holding means.

The ultraviolet transmitting section disposed at the opening portion on the lower side of the housing includes an ultraviolet transmitting plate and ultraviolet transmitting plate-holding means. A shape of the ultraviolet transmitting plate is not specifically limited although the shape depends on the shape of the ultraviolet lamp, and favorable examples of the shape can include a plate shape that is, e.g., a rectangular shape, a square shape, a round shape or an oval shape in plan view and a projecting shape that enables collection of ultraviolet light onto a sterilization target, and also can include, from the perspective of ensuring a certain length of time for sterilizing a sterilization target, a rectangular plate shape with the direction of movement of the sterilization target as a longitudinal direction thereof so as to conform to the long linear tube shape or the long U-tube shape of the ultraviolet lamp. A thickness of the ultraviolet transmitting plate is not specifically limited as long as the ultraviolet transmitting plate allows sterilization using the ultraviolet lamp; however, the thickness is preferably 0.5 to 15 mm, more preferably 2 to 7 mm, from the perspective of a balance between a strength of the ultraviolet transmitting plate and an ultraviolet light attenuation rate of the ultraviolet transmitting plate. A material of the ultraviolet transmitting plate is not specifically limited as long as the material can transmit ultraviolet light; however, examples of the material can include one described in Japanese Unexamined Patent Application Publication (Translation of PCT Application) No. 2005-519816 (Patent Document 3) in addition to quartz glass, and quartz glass is preferable because of its high ultraviolet transmittance. Also, in the present invention, it is preferable to provide one ultraviolet transmitting plate, but two ultraviolet transmitting plates may be provided unless the ultraviolet attenuation rate becomes excessively high. An enclosed space can also be provided at an outer peripheral part of a second ultraviolet transmitting plate, but may not be provided.

The ultraviolet transmitting plate-holding means is not specifically limited as long as the ultraviolet transmitting plate-holding means can hold the ultraviolet transmitting plate via the outer peripheral edge part thereof so as to be able to irradiate a sterilization target with the ultraviolet lamp, and comprises a sealing means for forming an enclosed space cooperating with the outer peripheral part of the ultraviolet transmitting plate. The ultraviolet transmitting plate-holding means may be a single-piece holding means including a recess groove for holding the outer peripheral edge part of the ultraviolet transmitting plate at each of three sides thereof, allowing the ultraviolet transmitting plate to be slid and thereby inserted into the holding recess grooves from an open end at another side; however, for ease of assembly and maintenance, a favorable example of the ultraviolet transmitting plate-holding means can be a holding means including a combination of a base plate (lower holding plate) and a retainer plate (upper holding plate) that can hold the ultraviolet transmitting plate therebetween.

An example of the base plate can be one that includes an opening portion at a part in which the ultraviolet transmitting plate is disposed, so as to be able to irradiate a sterilization target with the ultraviolet lamp and can hold the ultraviolet transmitting plate at an edge part of the opening portion cooperating with the retainer plate, and a favorable example of the base plate can be one including an opening edge part including a recessed step portion that allows the ultraviolet transmitting plate to be placed thereon, the recessed step portion having a depth that is larger than the thickness of the ultraviolet transmitting plate. Also, from the perspective of avoiding the risk of a drop of the ultraviolet transmitting plate, a more favorable example of the base plate can be one including an opening portion having a shape that is substantially similar to but smaller than the plan view shape of the ultraviolet transmitting plate. The opening portion of the base plate can include an opening end at a position that is 2 to 100 mm, preferably 5 to 50 mm, inside the outer peripheral end of the ultraviolet transmitting plate. For example, an opening portion including an opening end at a position that is 30 mm inside an outer peripheral end of an ultraviolet transmitting plate having a rectangular shape of 30 cm long and 60 cm wide in plan view has a rectangular shape of 24 cm long and 54 cm wide.

The retainer plate is not specifically limited as long as the retainer plate is one that includes an opening portion at a part in which the ultraviolet transmitting plate is disposed, so as to be able to irradiate a sterilization target with the ultraviolet lamp, and can hold the ultraviolet transmitting plate via an opening edge part thereof cooperating with the base plate; however, from the perspective of obtaining an enclosed space in a better sealed state, a favorable example of the retainer plate can be one that can press and hold the outer peripheral edge part of the ultraviolet transmitting plate cooperating with the base plate, and in particular, a more favorable example of the retainer plate can be one that can press and hold all outer peripheral edge parts of the ultraviolet transmitting plate cooperating with the base plate. A more favorable example of the opening portion of the retainer plate can be one having a shape that is substantially similar to but smaller than the plan view shape of the ultraviolet transmitting plate, in particular, one including an opening end at a position that is 2 to 100 mm, preferably 5 to 50 mm inside the outer edge of the ultraviolet transmitting plate is preferable.

Examples of the sealing means provided in the holding means can include a sealant such as a resin adhesive that can seal surfaces of. e.g., the base plate and the retainer plate that face each other, directly or via the ultraviolet transmitting plate, and a recess peripheral groove provided in the holding means and an elastic body such as nitrile rubber, styrene-butadiene rubber, silicone rubber or fluoro-rubber fitted in the recess peripheral groove; however, from the perspective of ease of assembly, a sealing means using an elastic body is preferable, and examples of a shape of the elastic body can include an 0-ring shape and a plate shape, but an 0-ring shaped elastic body (hereinafter referred to as "0-ring") is particularly preferable. Use of an 0-ring having a diameter that is a little larger than a depth of the recess groove portion enables provision of a better sealed state.

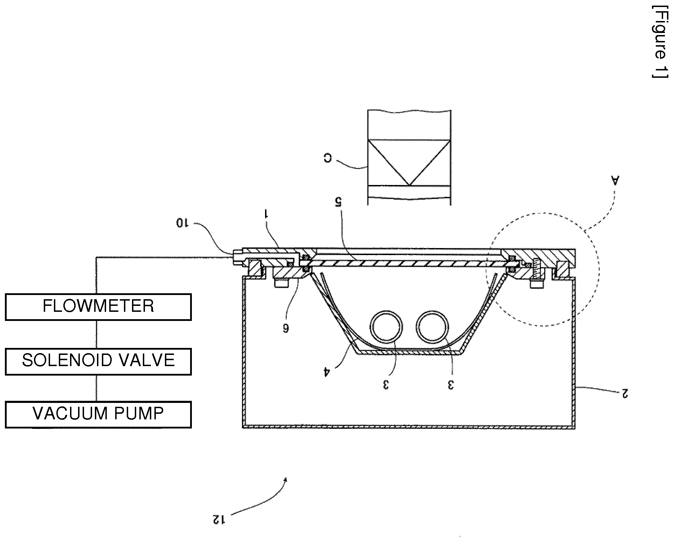

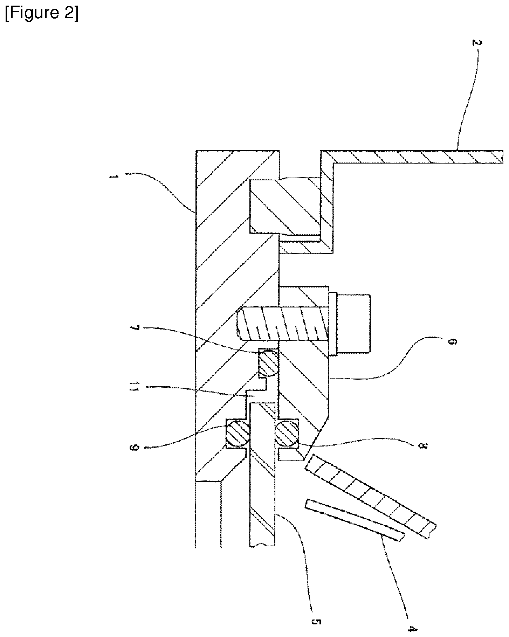

Examples of a sealing mode of the holding means including a combination of the base plate and the retainer plate can include a mode in which a part of the base plate and a part of the retainer plate which are outward of the outer peripheral end surface of the ultraviolet transmitting plate are directly sealed and the base plate and the retainer plate are sealed via the outer peripheral edge part of the ultraviolet transmitting plate (first sealing mode: see Figures 1 and 2) for forming an enclosed space outside the outer peripheral end surface of the ultraviolet transmitting plate along the outer peripheral end surface of the ultraviolet transmitting plate cooperating with the outer peripheral end surface of the ultraviolet transmitting plate, and a mode in which the base plate and the retainer plate are sealed via two areas in an upper surface side and/or a lower surface side of the outer peripheral edge part of the ultraviolet transmitting plate, the two parts having a predetermined interval therebetween (second sealing mode) for forming an enclosed space as a recess peripheral groove of the retainer plate that corresponds to the upper surface side of the outer peripheral edge part of the ultraviolet transmitting plate and/or a recess peripheral groove of the base plate that corresponds to the lower surface side of the ultraviolet transmitting plate cooperating with the upper surface and the lower surface of the outer peripheral edge part of the ultraviolet transmitting plate.

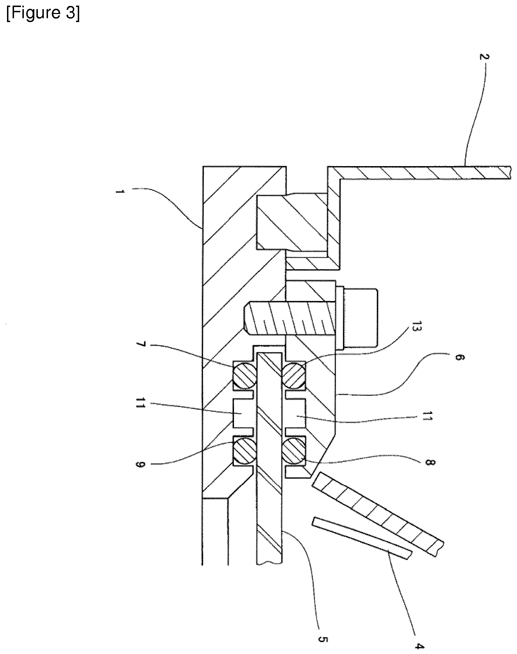

Examples of the second sealing mode can include a mode in which the base plate and the retainer plate are sealed via two parts on each of the upper surface side and the lower surface side of the outer peripheral edge part of the ultraviolet transmitting plate, the two parts having a predetermined interval therebetween, to form an enclosed space including two recess peripheral grooves, which are upper and lower recess peripheral grooves, each interposed between sealing means in the two parts of the retainer plate that corresponds to the upper surface side of the outer peripheral edge parts of the ultraviolet transmitting plate or the base plate that corresponds to the lower surface side of the outer peripheral edge part of the ultraviolet transmitting plate (see Figure 3) and a mode in which the base plate and the retainer plate are sealed via two parts on the upper surface side or the lower surface side of the outer peripheral edge part of the ultraviolet transmitting plate, the two parts having a predetermined interval therebetween, to form an enclosed space including one recess peripheral groove interposed between sealing means in the two parts of the retainer plate that corresponds to the upper surface side of the outer peripheral edge part of the ultraviolet transmitting plate or the base plate that corresponds to the lower surface side of the outer peripheral edge part of the ultraviolet transmitting plate; however, where this enclosed space including one recess peripheral groove is formed, on a surface of the base plate or the retainer plate that faces the retainer plate or the base plate on which the enclosed space including one recess peripheral groove is not formed, for example, two O-rings may be used, but one rubber plate can also be used.

The enclosed space formed along the outer side of the outer peripheral end surface of the ultraviolet transmitting plate and the enclosed space formed as a recess peripheral groove of the retainer plate that corresponds to the upper surface side of the outer peripheral edge part of the ultraviolet transmitting plate and/or the base plate that corresponds to the lower surface side of the same do not need to be formed around the entire outer peripheral part of the ultraviolet transmitting plate; however, from the perspective of detecting breakage of the ultraviolet transmitting plate with higher accuracy, it is preferable that the enclosed space be formed in a tubular shape around the entire outer peripheral part of the ultraviolet transmitting plate. An end of such enclosed space is in communication with air flow detecting means via a port (opening portion) and a piping, and such port can be called a vacuum port where air is suctioned from the enclosed space, and can be called a discharge port where air is introduced into the enclosed space.

The air flow detecting means is not specifically limited as long as the air flow detecting means includes an air pressure-adjusting means for adjusting an air pressure inside the enclosed space and a flowmeter, and the air pressure-adjusting means can introduce air having a predetermined pressure to the enclosed space or suction air having a predetermined pressure from the enclosed space to maintain the enclosed space at a positive pressure or a negative pressure. Examples of the air pressure-adjusting means include an air supply means for introducing air having a predetermined pressure to maintain the enclosed space in the ultraviolet transmitting section in a positive pressure state and an air suction means (a vacuum suction means) for suctioning air to maintain the enclosed space in the ultraviolet transmitting section at a negative pressure state.

The flowmeter is provided in, e.g., the piping between the air pressure-adjusting means and the enclosed space, and can detect a flow of air inside the enclosed space, the flow being generated when the enclosed space being released from the sealed state due to breakage of the ultraviolet transmitting plate, by detecting a flow of air inside the piping. It is preferable that a solenoid valve that can open or close the piping be disposed in the piping between the air pressure-adjusting means and the flowmeter. Maintaining the enclosed space in a negative pressure state is favorable in suppressing fly of broken pieces of the ultraviolet transmitting plate to the sterilization target side. Furthermore, maintaining the enclosed space at a negative pressure is preferable also in eliminating the need to provide a regulator and/or a pressure switch that are preferably provided in the piping where the enclosed space is maintained at a positive pressure. The regulator is a device that where the enclosed space is maintained at a positive pressure, controls a pressure supplied from the air supply means to provide an intended pressure, and the pressure switch is a switch for detecting an abnormality in pressure due to, e.g., a failure of the regulator and shutting off air supply. Where the regulator is installed in the piping, the regulator is preferably installed between the air pressure-adjusting means and the flowmeter, and where the solenoid valve is additionally used, the regulator is preferably installed between the air pressure-adjusting means and the solenoid valve. Where the pressure switch is installed in the piping, the pressure switch is preferably installed between the air pressure-adjusting means and the flowmeter, and where the regulator is additionally used, the pressure switch is preferably installed between the regulator and the flowmeter, and also, where the regulator and the solenoid valve are additionally used, the air pressure-adjusting means, the regulator, the solenoid valve, the pressure switch and the flowmeter are preferably arranged in this order. Here, the term "air" means not only air, but also any gas, including inert gases.

Where the enclosed space is in a positive pressure state, a gauge pressure (pressure index with an atmospheric pressure as a zero origin) in the enclosed space can be within a range of, for example, more than 0 kPaG and no more than 700 kPaG; however, from the perspective of detecting breakage of the ultraviolet transmitting plate with higher accuracy and also from the safety problem of, upon breakage of the ultraviolet transmitting plate, broken pieces flying because of the positive pressure, the gauge pressure is preferably within a range of 10 to 100 kPaG, more preferably within a range of 10 to 50 kPaG. On the other hand, where the enclosed space is in a negative pressure state, the gauge pressure of the enclosed space can be within a range of, for example, less than 0 kPaG to -101.3 kPaG, preferably within a range of -50 to -100 kPaG, more preferably within a range of -90 to -100 kPaG, from the perspective of detecting breakage of the ultraviolet transmitting plate with higher accuracy.

The flowmeter is not specifically limited as long as the flowmeter is one that, upon a flow of air occurs in the piping, can detect the flow of air; however, the flowmeter is preferably a flowmeter that can detect a flow of a slight amount of air, and it is preferable to use, for example, a flowmeter that can detect a minimum flow rate of around 0.05 to 0.5 L/min. More specifically, it is preferable that, for example, where a flowmeter that can detect a minimum flow rate of 0.2 L/min is used, the flow rate be set to the minimum flow rate so that upon a flow of air with a flow rate of no less than 0.2 L/min occurs, the flowmeter can detect such flow. A preferable example of such sensitive flowmeter can be a flow sensor (flow sensor-type flowmeter).

1 An ultraviolet sterilizer according to the present invention will be described below with reference to the drawings, but the present invention is not limited to the ultraviolet sterilizer. Figure 1 is a schematic cross-sectional view of an ultraviolet sterilizer according to the present invention, the ultraviolet sterilizer including sealing means of the first mode, Figure 2 is an enlarged view of part A in Figure 1. Figure 3 is a partial enlarged view of an ultraviolet sterilizer according to the present invention, the ultraviolet sterilizer including sealing means of the second mode.

1 Figures 1 and 2 illustrate an ultraviolet sterilizer according to the present invention (type I). This type of ultraviolet sterilizer comprises: a U-tube type (single lamp-type) ultraviolet lamp 3; a reflective plate 4; a housing 2 surrounding the ultraviolet lamp 3; an ultraviolet transmitting section provided at an opening portion on the lower side of the housing 2, the ultraviolet transmitting section including quartz glass 5 (ultraviolet transmitting plate) having a rectangular shape in plan view and a holding means for holding the quartz glass 5, the holding means including a base plate 1 and a retainer plate 6 fixed to the base plate 1 via screws; and an air flow detecting means including a vacuum pump (a air suction means), a solenoid valve and a flowmeter, and the base plate 1 includes a recess step for placing the quartz glass 5 thereon, and an opening in a roughly center portion, the opening having a rectangular shape that is similar to but smaller than a shape of the quartz glass 5, and when the quartz glass 5 is placed on a horizontal surface of the recess step, a tubular enclosed space is formed by four surfaces, which are an outer peripheral end surface of the ultraviolet transmitting plate, the horizontal surface of the recess step, a vertical surface of the recess step and a lower surface of the retainer plate 6, that is, a tubular enclosed space is formed cooperating with the outer peripheral end surface of the ultraviolet transmitting plate so as to extend along the outer side of the outer peripheral end surface of the ultraviolet transmitting plate and communicate with the air flow detecting means at an end thereof. Furthermore, for enhancement in sealing performance of the enclosed space, an 0-ring 7 is held between the retainer plate 6 and the base plate 1, an O-ring 8 is held between the retainer plate 6 and the ultraviolet transmitting plate, and an 0-ring 9 is held between the base plate 1 and the ultraviolet transmitting plate. The tubular enclosed space communicates with the air flow detecting means at the end thereof via a vacuum port 10, enabling the flowmeter to detect a flow of air in the tubular enclosed space generated when the tubular enclosed space is released from its sealed state due to breakage of the quartz glass 5.

1 In the ultraviolet sterilizer, the opening portion of the base plate 1 includes an opening end at a position that is 9.5 mm inside the outer edge of the quartz glass 5, and an opening portion of the retainer plate 6 includes an opening end at a position that is 7 mm inside the outer peripheral end of the quartz glass 5. Also, a recess peripheral groove for the 0-ring 7 is provided at a position that is around 3 mm from the outer peripheral end of the quartz glass 5, but may be provided at a position that is around 2.5 to 50 mm from the outer peripheral end of the quartz glass 5. A recess peripheral groove for the 0-ring 9 is provided at a position that is around 2 mm from the outer peripheral end of the quartz glass 5, but may be provided at a position that is around 0.5 to 45 mm from the outer peripheral end of the quartz glass 5.

Sterilization of a container by the ultraviolet sterilizer according to the present invention (type I) is performed, for example, as follows. While a gable top packaging container C is transported with a top portion thereof opened, under the ultraviolet lamp 3 in a longitudinal direction of the ultraviolet lamp, ultraviolet light provided from the ultraviolet lamp 3 (for example, a low-pressure mercury lamp) is maintained to have a high illuminance by a large reflective plate 4, ensuring application of the ultraviolet light to a bottom portion of the packaging container C for sterilization.

In the above ultraviolet sterilizer, upon the solenoid valve being opened, air inside the tubular enclosed space 11 is suctioned by the vacuum pump via the vacuum port 10 and a piping, whereby the inside of the enclosed space 11 is maintained in a predetermined negative pressure state. During the sealed state of the tubular enclosed space 11 being maintained, there is no flow of air in the piping; however, upon breakage of a part of the quartz glass 5, air flows into the tubular enclosed space 11 and a flow of air generates inside the piping, and the flowmeter detects the flow of air. In the ultraviolet sterilizer according to the present invention, the quartz glass 5 is maintained in a flexible state in which the quartz glass 5 is sandwiched by O-rings, that is, the ultraviolet transmitting plate is maintained in a state in which the ultraviolet transmitting plate is not in direct contact with the retainer plate and the base plate. Therefore, generation of even a fine break, such as a fine crack, causes deformation of the quartz glass 5, the crack reaches an end part of the quartz glass 5, air flows into the tubular enclosed space, the flow of air causes a flow of air inside the piping, and the flowmeter detects the flow, whereby the breakage of the quartz glass 5 can be detected with higher accuracy.

Figure 3 is a partial enlarged view of an ultraviolet sterilizer according to the present invention (type II). A base plate 1 and a retainer plate 6 each include an opening portion at a roughly center portion, the opening portion having a rectangular shape that is similar to but smaller than a shape of quartz glass 5, and in opening edge parts of the base plate 1 and the retainer plate 6, that is, a part of the base plate that corresponds to the lower surface side of an outer peripheral edge part of the ultraviolet transmitting plate and a part of the retainer plate that corresponds to the upper surface side of the ultraviolet transmitting plate, respective recess peripheral grooves for 0-rings 7 and 13 and respective recess peripheral grooves for 0-rings 9 and 8 are provided with respective enclosed spaces 11 therebetween.

In such ultraviolet sterilizer, the opening portion of the base plate 1 includes an opening end at a position that is 19.5 mm inside the outer edge of the quartz glass 5, and the opening portion of the retainer plate 6 includes an opening end at a position that is 17 mm inside the outer peripheral end of the quartz glass 5. Also, each of the recess peripheral grooves for the 0-rings 7 and 13 is provided at a position that is around 2 mm from the outer peripheral end of the quartz glass 5, and each of the recess peripheral grooves for the 0-rings 9 and 8 is provided at a position that is around 12 mm from the outer peripheral end of the quartz glass 5.

### Industrial Applicability

An ultraviolet sterilizer according to the present invention can be used for sterilizing, e.g., a food packaging container.

### Explanation of Letters or Numerals

- 1: base plate
- 2: housing
- 3: ultraviolet lamp
- 4: reflective plate
- 5: quartz glass
- 6: retainer plate
- 7: O-ring
- 8: O-ring
- 9: O-ring
- 10: vacuum port
- 11: tubular enclosed space
- 12: ultraviolet sterilizer
- 13: O-ring (fourth annular elastic body)
- C: packaging container

## Claims

1. An ultraviolet sterilizer (12) comprising: an ultraviolet lamp (3); a housing (2) surrounding the ultraviolet lamp (3); an ultraviolet transmitting section provided at an opening portion on a lower side of the housing (2), the ultraviolet transmitting section including an ultraviolet transmitting plate (5) and an ultraviolet transmitting plate(5)-holding means(1,6); and an air flow detecting means, **characterized in that**
the ultraviolet transmitting section-holding means (1,6) comprises a sealing means for forming an enclosed space (11) which one end is in communication with the air flow detecting means, cooperating with an outer peripheral part of the ultraviolet transmitting plate (5),
the air flow detecting means comprises an air pressure-adjusting means for adjusting an air pressure inside the enclosed space (11) and a flowmeter, and
wherein a flow of air inside the enclosed space (11) generated when the enclosed space (11) is released from a sealed state due to breakage of the ultraviolet transmitting plate (5) is detected by the flowmeter.

2. The ultraviolet sterilizer (12) according to claim 1, wherein the outer peripheral part of the ultraviolet transmitting plate (5) is an outer peripheral end surface or an outer peripheral edge part of the ultraviolet transmitting plate (5).

3. The ultraviolet sterilizer (12) according to claim 1 or 2, wherein the ultraviolet transmitting plate (5)-holding means (1,6) comprises a base plate (1) fixed to a lower end of a side wall of the housing (2), and a retainer plate (6) fixed on the base plate (1).

4. The ultraviolet sterilizer (12) according to claim 3, wherein the sealing means is a means for directly sealing a part of the base plate (1) and a part of the retainer plate (6) which are outward of the outer peripheral end surface of the ultraviolet transmitting plate (5); and sealing the base plate (1) and the retainer plate (6) via an outer peripheral edge part of the ultraviolet transmitting plate (5), and wherein an enclosed space (11) is formed along an outer side of the outer peripheral end surface of the ultraviolet transmitting plate (5).

5. The ultraviolet sterilizer (12) according to claim 3, wherein the sealing means is a means for sealing the base plate (1) and the retainer plate (6) via two parts in the outer peripheral edge part of the ultraviolet transmitting plate (5), the two parts having a predetermined interval therebetween, and an enclosed space (11) is formed as a recess peripheral groove of the retainer plate (6) that corresponds to an upper surface side of the outer peripheral edge portion of the ultraviolet transmitting plate (5) and/or a recess peripheral groove of the base plate (1) that corresponds to a lower surface side of the outer peripheral edge part of the ultraviolet transmitting plate (5).

6. The ultraviolet sterilizer (12) according to any one of claims 1 to 5, wherein the sealing means is an elastic body (7,8,9,13) having an O-ring shape.

7. The ultraviolet sterilizer (12) according to any one of claims 1 to 6, wherein the air pressure adjusting means is an air suction means for suctioning air having a predetermined pressure from the enclosed space (11) to maintain the enclosed space (11) in a negative pressure state.

8. The ultraviolet sterilizer (12) according to any one of claims 1 to 7, wherein the ultraviolet transmitting plate (5) is made of quartz glass.

## Patentansprüche

1. Ultraviolettsterilisator (12), welcher umfasst:
eine Ultraviolettlampe (3); ein Gehäuse (2), das die Ultraviolettlampe (3) umgibt; einen Ultraviolett-Übertragungsabschnitt, der an einem Öffnungsabschnitt auf einer Unterseite des Gehäuses (2) vorgesehen ist, wobei der Ultraviolett-Übertragungsabschnitt eine Ultraviolett-Übertragungsplatte (5) und ein Haltemittel (1, 6) für die Ultraviolett-Übertragungsplatte (5) aufweist; und ein Luftstrom-Erkennungsmittel, **dadurch gekennzeichnet, dass**
das Haltemittel (1, 6) des Ultraviolett-Übertragungsabschnitts ein Dichtmittel zum Ausbilden eines umschlossenen Raumes (11) umfasst, von dem ein Ende in Kommunikation mit dem Luftstrom-Erkennungsmittel steht, wobei es mit einem äußeren Umfangsteil der Ultraviolett-Übertragungsplatte (5) zusammenwirkt,
das Luftstrom-Erkennungsmittel ein Luftdruck-Einstellmittel zum Einstellen eines Luftdrucks innerhalb des umschlossenen Raumes (11) und einen Durchflussmesser umfasst, und
wobei ein Strom von Luft innerhalb des umschlossenen Raumes (11), der erzeugt wird, wenn ein abgedichteter Zustand des umschlossenen Raumes (11) infolge eines Bruchs der Ultraviolett-Übertragungsplatte (5) aufgehoben wird, von dem Durchflussmesser erkannt wird.

2. Ultraviolettsterilisator (12) nach Anspruch 1, wobei der äußere Umfangsteil der Ultraviolett-Übertragungsplatte (5) eine Außenumfangs-Stirnfläche oder ein Außenumfangs-Randteil der Ultraviolett-Übertragungsplatte (5) ist.

3. Ultraviolettsterilisator (12) nach Anspruch 1 oder 2, wobei das Haltemittel (1, 6) für die Ultraviolett-Übertragungsplatte (5) eine Grundplatte (1), die an einem unteren Ende einer Seitenwand des Gehäuses (2) befestigt ist, und eine Halteplatte (6), die auf der Grundplatte (1) befestigt ist, umfasst.

4. Ultraviolettsterilisator (12) nach Anspruch 3, wobei das Dichtmittel ein Mittel zum direkten Abdichten eines Teils der Grundplatte (1) und eines Teils der Halteplatte (6), welche außenliegend bezüglich der Außenumfangs-Stirnfläche der Ultraviolett-Übertragungsplatte (5) sind, und Abdichten der Grundplatte (1) und der Halteplatte (6) über einen Außenumfangs-Randteil der Ultraviolett-Übertragungsplatte (5) ist, und wobei ein umschlossener Raum (11) entlang einer Außenseite der Außenumfangs-Stirnfläche der Ultraviolett-Übertragungsplatte (5) ausgebildet ist.

5. Ultraviolettsterilisator (12) nach Anspruch 3, wobei das Dichtmittel ein Mittel zum Abdichten der Grundplatte (1) und der Halteplatte (6) über zwei Teile in dem Außenumfangs-Randteil der Ultraviolett-Übertragungsplatte (5) ist, wobei die zwei Teile einen vorbestimmten Abstand voneinander aufweisen und ein umschlossener Raum (11) als eine ausgesparte Umfangsnut der Halteplatte (6) ausgebildet ist, welche einer oberen Oberflächenseite des Außenumfangs-Randabschnitts der Ultraviolett-Übertragungsplatte (5) entspricht, und/oder als eine ausgesparte Umfangsnut der Grundplatte (1), welche einer unteren Oberflächenseite des Außenumfangs-Randteils der Ultraviolett-Übertragungsplatte (5) entspricht.

6. Ultraviolettsterilisator (12) nach einem der Ansprüche 1 bis 5, wobei das Dichtmittel ein elastischer Körper (7, 8, 9, 13) mit einer O-RingForm ist.

7. Ultraviolettsterilisator (12) nach einem der Ansprüche 1 bis 6, wobei das Luftdruck-Einstellmittel ein Luftabsaugmittel zum Absaugen von Luft mit einem vorbestimmten Druck aus dem umschlossenen Raum (11), um den umschlossenen Raum (11) in einem Unterdruckzustand zu halten, ist.

8. Ultraviolettsterilisator (12) nach einem der Ansprüche 1 bis 7, wobei die Ultraviolett-Übertragungsplatte (5) aus Quarzglas hergestellt ist.

## Revendications

1. Stérilisateur ultraviolet (12) comprenant : une lampe ultraviolette (3) ; un boîtier (2) entourant la lampe ultraviolette (3) ; une section transmettant les ultraviolets disposée dans une partie d'ouverture sur un côté inférieur du boîtier (2), la section transmettant les ultraviolets comportant une plaque transmettant les ultraviolets (5) et un moyen de support (1, 6) de la plaque transmettant les ultraviolets (5) ; et un moyen de détection d'écoulement d'air, **caractérisé en ce que**
le moyen de support de plaque transmettant les ultraviolets (1, 6) comprend un moyen de scellement pour former un espace fermé (11) dont une extrémité est en communication avec le moyen de détection d'écoulement d'air, coopérant avec une partie périphérique extérieure de la plaque transmettant les ultraviolets (5),
le moyen de détection d'écoulement d'air comprend un moyen d'ajustement de pression d'air pour ajuster une pression d'air à l'intérieur de l'espace fermé (11) et un débitmètre, et
dans lequel un écoulement d'air à l'intérieur de l'espace fermé (11) généré quand l'espace fermé (11) est libéré d'un état scellé en raison d'une rupture de la plaque transmettant les ultraviolets (5) est détecté par le débitmètre.

2. Stérilisateur ultraviolet (12) selon la revendication 1, dans lequel la partie périphérique extérieure de la plaque transmettant les ultraviolets (5) est une surface d'extrémité périphérique extérieure ou une partie de bord périphérique extérieur de la plaque transmettant les ultraviolets (5).

3. Stérilisateur ultraviolet (12) selon la revendication 1 ou 2, dans lequel le moyen de support (1, 6) de la plaque transmettant les ultraviolets (5) comprend une plaque de base (1) fixée à une extrémité inférieure d'une paroi latérale du boîtier (2), et une plaque de retenue (6) fixée sur la plaque de base (1).

4. Stérilisateur ultraviolet (12) selon la revendication 3, dans lequel le moyen de scellement est un moyen pour sceller directement une partie de la plaque de base (1) et une partie de la plaque de retenue (6) qui sont à l'extérieur de la surface d'extrémité périphérique extérieure de la plaque transmettant les ultraviolets (5) ; et sceller la plaque de base (1) et la plaque de retenue (6) par le biais d'une partie de bord périphérique extérieur de la plaque transmettant les ultraviolets (5), et dans lequel un espace fermé (11) est formé le long d'un côté extérieur de la surface d'extrémité périphérique extérieure de la plaque transmettant les ultraviolets (5).

5. Stérilisateur ultraviolet (12) selon la revendication 3, dans lequel le moyen de scellement est un moyen pour sceller la plaque de base (1) et la plaque de retenue (6) par le biais de deux parties dans la partie de bord périphérique extérieur de la plaque transmettant les ultraviolets (5), les deux parties ayant un intervalle prédéterminé entre elles, et un espace fermé (11) est formé comme une rainure périphérique en retrait de la plaque de retenue (6) qui correspond à un côté de surface supérieure de la partie de bord périphérique extérieur de la plaque transmettant les ultraviolets (5) et/ou une rainure périphérique en retrait de la plaque de base (1) qui correspond à un côté de surface inférieure de la partie de bord périphérique extérieur de la plaque transmettant les ultraviolets (5).

6. Stérilisateur ultraviolet (12) selon l'une quelconque des revendications 1 à 5, dans lequel le moyen de scellement est un corps élastique (7, 8, 9, 13) ayant une forme de joint torique.

7. Stérilisateur ultraviolet (12) selon l'une quelconque des revendications 1 à 6, dans lequel le moyen d'ajustement de pression d'air est un moyen d'aspiration d'air pour aspirer de l'air ayant une pression prédéterminée depuis l'espace fermé (11) pour maintenir l'espace fermé (11) dans un état de pression négative.

8. Stérilisateur ultraviolet (12) selon l'une quelconque des revendications 1 à 7, dans lequel la plaque transmettant les ultraviolets (5) est constituée de verre de quartz.
